(19) Europäisches Patentamt
European Patent Office
Office européen des brevets

(11) Veröffentlichungsnummer: **0 137 426**
**A2**

(12) # EUROPÄISCHE PATENTANMELDUNG

(21) Anmeldenummer: **84111599.1**

(22) Anmeldetag: **28.09.84**

(51) Int. Cl.⁴: **C 07 D 277/36**
C 07 D 277/72, A 61 K 31/425

(30) Priorität: **11.10.83 DE 3336846**

(43) Veröffentlichungstag der Anmeldung:
**17.04.85 Patentblatt 85/16**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Oediger, Hermann, Dr.**
**Roggendorfstrasse 51**
**D-5000 Köln 80(DE)**

(72) Erfinder: **Lieb, Folker, Dr.**
**Alfred-Kubin-Strasse 1**
**D-5090 Leverkusen 1(DE)**

(72) Erfinder: **Perzborn, Elisabeth, Dr.**
**Am Tescherbusch 13**
**D-5600 Wuppertal(DE)**

(72) Erfinder: **Seuter, Friedel, Dr.**
**Moospfad 16**
**D-5600 Wuppertal 1(DE)**

(54) **Neue 2-Mercaptothiazolderivate, Verfarhen zu deren Herstellung sowie die Verwendung von 2-Mercaptothiazolderivaten in Arzneimitteln.**

(57) Die vorliegende Erfindung betrifft neue 2-Mercaptothiazolderivate, Verfahren zu ihrer Herstellung sowie die Verwendung von 2-Mercaptothiazolderivaten in Arzneimitteln, insbesondere in thromboembolischen Mitteln.

EP 0 137 426 A2

Croydon Printing Company Ltd.

0137426

BAYER AKTIENGESELLSCHAFT 5090 Leverkusen, Bayerwerk

Konzernverwaltung RP
Patentabteilung Sft/Em-c

Neue 2-Mercaptothiazolderivate, Verfahren zu deren Herstellung sowie die Verwendung von 2-Mercaptothiazolderivaten in Arzneimitteln

Die vorliegende Erfindung betrifft neue 2-Mercaptothiazolderivate, Verfahren zu ihrer Herstellung sowie die Verwendung von 2-Mercaptothiazolderivaten in Arzneimitteln, insbesondere in Antithrombotika, Antiasthmatika und antiischämischen Mitteln.
Thrombose und arteriosklerotische Gefäßveränderungen werden vor allem durch die Wechselwirkung zweier Metaboliten der Arachidonsäure, nämlich durch das Thromboxan $A_2$ ($TXA_2$) und auf der anderen Seite durch das Prostacyclin ($PGI_2$) gesteuert. $TXA_2$ wirkt auf die Blutplättchen aggregierend, und $PGI_2$ hat eine antiaggregierende Wirkung. Darüber hinaus wirkt $TXA_2$ vasokonstriktorisch und $PGI_2$ vasodilatatorisch.

Bei einer Reihe von thrombo-embolischen und ischämischen Erkrankungen führt Hyperaggregabilität der Plättchen bzw. ein erhöhter Plättchenverbrauch zu einer gesteigerten Thromboxansynthese, so daß das $TXA_2$- und $PGI_2$-Gleichgewicht gestört ist. Es ist deshalb zur Therapie und

Prophylaxe von thrombo-embolischen und ischämischen Erkrankungen wünschenswert, die Thromboxanwirkung zu hemmen und damit die protektive Eigenschaft des $PGI_2$ zu erhöhen.

Es wurde nunmehr überraschenderweise gefunden, daß bestimmte 2-Mercaptothiazolderivate eine starke und spezifische antagonistische Wirkung bezüglich Thromboxan $A_2$ aufweisen.

Gegenstand der Erfindung sind Thromboxan-antagonistische und plättchenaggregationshemmende Arzneimittel, dadurch gekennzeichnet, daß sie 2-Mercaptothiazolderivate der allgemeinen Formel

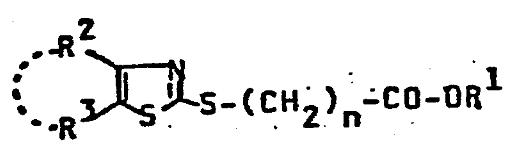

(I)

oder deren pharmakologisch unbedenkliche Salze enthalten, in welcher

$R^1$ für Wasserstoff oder einen gegebenenfalls verzweigten Alkylrest mit 1 bis 6 C-Atomen steht,

n eine ganze Zahl von 2 bis 10 bedeutet und

$R^2$ und $R^3$ gleich oder verschieden sind und für Wasserstoff, Halogen, Cyano, Amino, einen gegebenenfalls verzweigten und gegebenenfalls durch Halogen, Hydroxy, Methoxy oder Carboxy substituierten Alkyl- oder Acylrest mit 1 bis 6 C-Atomen oder einen Arylrest mit 6 bis 10 C-Atomen, welcher gegebenenfalls durch Halogen, $CF_3$, Nitro, $C_1$-$C_6$-Alkyl, $C_1$-$C_6$-Alkoxy, Carboxy, Amino, Alkylamino oder Dialkylamino mit 1 bis 4 C-Atomen in den Alkylgruppen, Acyl mit 1 bis 6 C-Atomen oder Acylamino mit 1 bis 6 C-Atomen oder einen Methylendioxyrest substituiert ist, stehen oder

$R^2$ und $R^3$ zusammen mit dem Thiazolring einen gegebenenfalls teilweise hydrierten aromatischen Ring mit 5 bis 7 C-Atomen bilden, welcher gegebenenfalls durch 1 oder 2 Substituenten aus der Gruppe Halogen, Hydroxy, Alkoxy mit 1 bis 6 C-Atomen, Amino, Alkylamino oder Dialkylamino mit 1 bis 4 C-Atomen in den Alkylgruppen, Carboxy, Nitro, Alkyl mit 1 bis 6 C-Atomen, Trifluormethyl, Acyl mit 1 bis 6 C-Atomen oder Acylamino mit 1 bis 6 C-Atomen, oder durch einen Methylendioxyrest substituiert ist.

Gegenstand der Erfindung sind auch Verbindungen der allgemeinen Formel

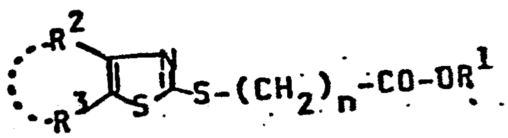

(I)

oder deren pharmakologisch unbedenkliche Salze, in welcher

$R^1$ für Wasserstoff oder einen gegebenenfalls verzweigten Alkylrest mit 1 bis 6 C-Atomen steht,

n eine ganze Zahl von 2 bis 10 bedeutet und

$R^2$ und $R^3$ gleich oder verschieden sind und für Wasserstoff, Halogen, Cyano, Amino, einen gegebenenfalls verzweigten und gegebenenfalls durch Halogen, Hydroxy, Methoxy oder Carboxy substituierten Alkyl- oder Acylrest mit 1 bis 6 C-Atomen oder einen Arylrest mit 6 bis 10 C-Atomen, welcher gegebenenfalls durch Halogen, $CF_3$, Nitro, $C_1$-$C_6$-Alkyl, $C_1$-$C_6$-Alkoxy, Carboxy, Amino, Alkylamino oder Dialkylamino mit 1 bis 4 C-Atomen in den Alkylgruppen, Acyl mit 1 bis 6 C-Atomen oder Acylamino mit 1 bis 6 C-Atomen oder einen Methylendioxyrest substituiert ist, stehen oder

$R^2$ und $R^3$ zusammen mit dem Thiazolring einen gegebenenfalls teilweise hydrierten aromatischen Ring mit 5 bis 7 C-Atomen bilden, welcher gegebenenfalls durch 1 oder 2 Substituenten aus der Gruppe Halogen, Hydroxy, Alkoxy mit 1 bis 6 C-Atomen, Amino, Alkylamino oder Dialkylamino mit 1 bis 4 C-Atomen in den Alkylgruppen, Carboxy, Nitro, Alkyl mit 1 bis 6 C-Atomen, Trifluormethyl, Acyl mit 1 bis 6 C-Atomen oder Acylamino mit 1 bis 6 C-Atomen, oder durch einen Methylendioxyrest substituiert ist,

mit der Maßgabe, daß $R^2$ und $R^3$ zusammen mit dem Thiazolring keinen unsubstituierten 6-gliedrigen aromatischen Ring bilden, wenn $R^1$ für Wasserstoff steht.

Die in den erfindungsgemäßen Arzneimitteln enthaltenen Verbindungen sind teilweise bekannt:

Izv. Akad. SSSR 1969, Heft Nr. 3, Seite 693

Aus EP-A 0 006 347 und JA-A 57059-871 ist die Verwendung bestimmter Thiazolderivate als Wachstumsregulatoren bzw. als entzündungshemmende Mittel bekannt.

Die nunmehr gefundenen speziellen pharmakologischen Wirkungen der oben definierten 2-Mercaptothiazolderivate sind jedoch neu.

Die Herstellung der erfindungsgemäßen Verbindungen erfolgt, indem man entweder

A) 2-Mercaptothiazole der allgemeinen Formel (II)

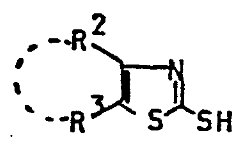

(II)

in welcher
$R^2$ und $R^3$ die oben angegebene Bedeutung haben,

mit ω-Halogencarbonsäureestern der allgemeinen Formel (III)

$$Hal-(CH_2)_n-CO-OR^1 \qquad (III)$$

in welcher

$R^1$ für einen gegebenenfalls verzweigten Alkylrest mit 1 bis 6 C-Atomen steht,

n die oben angegebene Bedeutung hat und

Hal für ein Halogenatom steht,

in Gegenwart einer Base und gegebenenfalls einem inerten organischen Lösungsmittel bei Temperaturen zwischen 0 und 150°C umsetzt und gegebenenfalls die so erhaltenen Verbindungen der allgemeinen Formel (I) mit $R^1 \neq H$ verseift

oder (für den Fall, daß n für die Zahl 2 steht),

B) 2-Mercaptothiazole der allgemeinen Formel (II) mit Vinylverbindungen der allgemeinen Formel (VI)

$$CH_2=CH-R^4 \qquad (VI)$$

in welcher
$R^4$ für CN oder $CO-OR^1$ steht,

wobei $R^1$ die obige Bedeutung hat, in Gegenwart eines basischen Katalysators und gegebenenfalls in einem inerten organischen Lösungsmittel bei Temperaturen zwischen 0 - 80°C umsetzt und für den Fall, daß $R^4$ für $CO-OR^1$ steht, gegebenenfalls die so erhaltenen Verbindungen der allgemeinen Formel (I) verseift oder für den Fall, daß $R^4$ für CN steht, die erhaltene Verbindung der allgemeinen Formel (V)

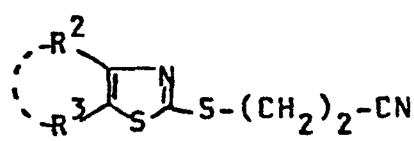

(V)

verseift.

Verwendet man im Verfahren A) als Ausgangsstoffe 2-Mercaptobenzthiazol und 4-Brombutansäuremethylester und als Base Natriumhydrid, so kann der Reaktionsablauf durch folgendes Formelschema wiedergegeben werden:

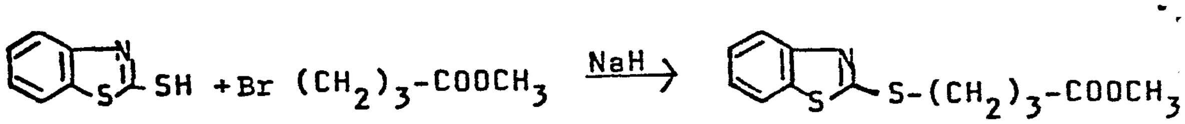


Verwendet man im Verfahren B) als Ausgangsstoffe 4,5-Dichlor-2-mercaptothiazol und Acrylnitril und als basichen Katalysator eine quartäre Ammoniumverbindung und zur Verseifung der Verbindung (V) Schwefelsäure, so kann der Reaktionsablauf durch folgendes Formelschema wiedergegeben werden:

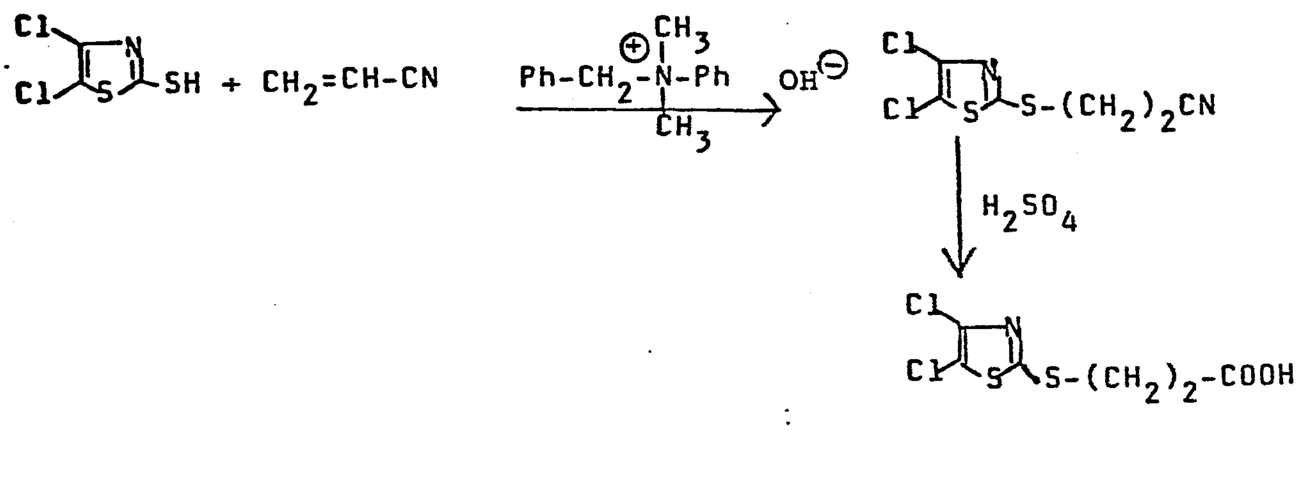

Die als Ausgangsstoffe verwendeten 2-Mercaptothiazole sind bekannt oder können nach bekannten Verfahren hergestellt werden.

(J. Am. Chem. Soc., Bd. 49, 1927, Seite 1748; US-Patent 2 186 419; J. Am. Chem. Soc.,Bd. 70, 1948, S. 1451; FR-Patentanmeldung 71.26844; EP-A 0.061.425; C. Ferri, Reaktionen der organischen Synthese, 1. Auflage, S. 736; Ullmanns Enzyklopädie der Technischen Chemie, 3. Auflage, Band XII, Seite 305; Chem. Zyesti, Band 27 (Nr. 5), 1973, S. 698).

In der allgemeinen Formel (II) stehen $R^2$ und $R^3$, die gleich oder verschieden sein können, vorzugsweise für Wasserstoff, für Halogen wie Fluor, Chlor, Brom, Jod (besonders für Chlor und Brom), für Cyano, für Acetyl, für geradkettiges oder verzweigtes Alkyl mit 1 bis 6 Kohlenstoffatomen, welches gegebenenfalls durch Halogen substituiert ist, für Phenyl, welches gegebenenfalls durch Halogen, Trifluormethyl, Hydroxy, Niedrigalkyloxy mit 1 bis 4 Kohlenstoffatomen oder Methylendioxy substituiert ist; für die Butadiengruppe, falls $R^2$ und $R^3$ zusammen mit dem Thiazolring einen aromatischen Ring bilden, welcher gegebenenfalls durch Halogen, Hydroxy, Alkoxy mit 1 bis 4 Kohlenstoffatomen, Amino, N,N-Dialkylamino, Nitro oder Carboxy substituiert ist; für eine Alkylengruppe, beispielsweise eine Propylen-, Butylen- oder Pentylengruppe, falls $R^2$ und $R^3$ zusammen mit dem Thiazolring einen teilweise hydrierten aromatischen Ring bilden.

Als 2-Mercaptothiazole der allgemeinen Formel (II) seien beispielhaft genannt:

2-Mercaptothiazol,
2-Mercapto-4-methylthiazol,
2-Mercapto-4-methyl-5-acetylthiazol,
2-Mercapto-4,5-dimethylthiazol,
2-Mercapto-4,5-dichlorthiazol,
2-Mercapto-4,5-dicyanothiazol,
2-Mercapto-4-chlor-5-cyanothiazol,
2-Mercapto-5-trifluormethylthiazol
2-Mercapto-5-aminothiazol
2-Mercapto-4-phenylthiazol,
2-Mercapto-4,5-diphenylthiazol,
2-Mercapto-4,5-bis(4'-methoxyphenyl)-thiazol,
2-Mercapto-4,5-bis(3',4'-methylendioxyphenyl)-thiazol,
2-Mercapto-benzothiazol,
2-Mercapto-5-chlor-benzothiazol,
2-Mercapto-4-fluor-benzothiazol,
2-Mercapto-6-fluor-benzothiazol,
2-Mercapto-5-fluor-benzothiazol,
2-Mercapto-7-fluor-benzothiazol,
2-Mercapto-5,6-difluorbenzthiazol,
2-Mercapto-4-ethoxy-6-chlor-benzothiazol,
2-Mercapto-5-ethoxy-benzothiazol,
2-Mercapto-6-dimethylamino-benzothiazol,
2-Mercapto-6-aminobenzothiazol,
2-Mercapto-6-nitro-benzothiazol,
2-Mercapto-5-carboxy-benzothiazol,
2-Mercapto-6-formamido-benzothiazol.

2-Mercapto-5,6-dihydro-4H-cyclopentathiazol
2-Mercapto-4,5,6,7-tetrahydro-benzothiazol
2-Mercapto-5,6,7,8-tetrahydro-4H-cycloheptathiazol.

Die im Verfahren A) ebenfalls als Ausgangsstoffe verwendeten ω-Halogencarbonsäureester der allgemeinen Formel (III) sind bekannt oder können nach bekannten Verfahren hergestellt werden. (C. r. Band 102, S. 369; Liebigs Ann. Chem. Bd. 319, S. 388; J. Am. Chem. Soc. Bd. 60, 1938, S. 1375).

In der allgemeinen Formel (III) steht vorzugsweise

$R^1$ für ein geradkettiges oder verzweigtes Alkyl mit 1 bis 4 Kohlenstoffatomen, insbesondere für Methyl oder Ethyl

Hal für Chlor, Brom oder Jod, insbesondere für Brom;

n für eine Zahl von 2 - 4, insbesondere für 3.

Als ω-Halogencarbonsäureester der allgemeinen Formel (III) seien beispielhaft genannt:

3-Chlorpropansäureethylester
4-Chlorbutansäuremethylester
4-Brombutansäuremethylester
4-Brombutansäureethylester
4-Brombutansäurepropylester
4-Brombutansäurebutylester
5-Brompentansäuremethylester
5-Brompentansäureethylester

Die im Verfahren A) verwendete Base kann eine anorganische oder organische Base sein. Als anorganische Base eignen sich z.B. Alkalihydroxide, vorzugsweise Natrium, oder Kaliumhydroxid oder Alkalihydride, vorzugsweise Natrium- oder Kaliumhydrid. Als organische Basen eignen sich beispielsweise Alkalialkoxide, vorzugsweise Natriummethoxid,

Kalium-tert.-butoxid, metallorganische Verbindungen wie Butyllithium, Phenyllithium oder auch tert. Amine, beispielsweise 1,4-Diazabicyclo[2.2.2]octan, 1,5-Diazabicyclo[4.3.0]non-5-en, oder 1,8-Diazabicyclo[5.4.0]undec-7-en.

Als Verdünnungsmittel kommen alle inerten organischen Lösungsmittel in Frage. Hierzu gehören vorzugsweise cyclische oder acyclische Ether wie Tetrahydrofuran, Dioxan oder Dimethoxyethan, aliphatische Carbonsäurenitrile wie Acetonitril oder Propionitril, aliphatische N-substituierte Carbonsäureamide wie Dimethylformamid oder Dimethylacetamid, Phosphorsäureamide wie Hexamethylphosphorsäurediamid und Sulfoxide wie Dimethylsulfoxid.

Die Reaktionstemperaturen können in einem größeren Bereich variiert werden. Im allgemeinen arbeitet man zwischen 0 und 150°C, vorzugsweise zwischen 20 und 80°C.

Bei der Duchführung des erfindungsgemäßen Verfahrens setzt man in der Regel 1 Mol der Verbindung (II) mit 1 bis 1,5 Mol, vorzugsweise 1,1 Mol der Verbindung (III) in Gegenwart von 1 bis 1,5 Mol, vorzugsweise 1 bis 1,1 Mol einer Base um.

Die Reaktionsdauer ist von der Temperatur abhängig und liegt in der Regel zwischen 30 min. und 10 Stunden.

Falls erforderlich, kann eine Reinigung entweder durch Umkristallisation, Destillation oder durch Chromatographie erfolgen.

Gegebenenfalls werden die erfindungsgemäß erhaltenen Verbindungen der allgemeinen Formel (I) in bekannter Weise verseift. Dafür kommen beispielsweise Alkalihydroxide wie insbesondere Natriumhydroxid oder Kaliumhydroxid in Form ihrer verdünnten wäßrigen (oder) alkoholischen wie methanolischen oder ethanolischen oder alkoholisch-wäßrigen Lösungen infrage. Es eignen sich aber auch verdünnte wäßrige Mineralsäuren, beispielsweise 2 n wäßrige Schwefelsäure in einem inerten organischen Lösungsmittel, beispielsweise Dioxan.

Die im Verfahren B) ebenfalls als Ausgangsstoffe verwendeten Vinylverbindungen der allgemeinen Formel (IV) sind bekannt.

In der allgemeinen Formel (IV) steht vorzugsweise

$R^1$ für einen geradkettigen oder verzweigten Alkylrest mit 1 bis 4 Kohlenstoffatomen, insbesondere für Methyl oder Ethyl.

Als ungesättigte Verbindungen der allgemeinen Formel (IV) seien beispielhaft genannt:
Acrylnitril, Acrylsäuremethylester, und Acrylsäureethylester.

Die im Verfahren B) verwendeten Katalysatoren können z.B. quartäre Ammoniumverbindungen sein. Beispielsweise sind geeignet:

0137426

N-Benzyl-N,N-dimethyl-aniliniumhydroxid, Benzyl-triethylammoniumhydroxid, Benzyl-dodecyldimethylammoniumhydroxid, und Benzyltrimethylammoniumhydroxid.

Als Verdünnungsmittel kommen wieder alle inerten organischen Lösungsmittel in Frage. Hierzu gehören vorzugsweise aromatische Kohlenwasserstoffe wie Toluol oder Xylol, cyclische oder acyclische Ether wie Tetrahydrofuran, Dioxan oder Dimethoxyethan, aliphatische Carbonsäurenitrile wie Acetonitril oder Propionitril, aliphatische N-substituierte Carbonsäureamide wie Dimethylformamid oder Dimethylacetamid, Phosphorsäureamide wie Hexamethylphosphorsäureamid und Sulfoxide wie Dimethylsulfoxid.

Insbesondere können auch die ungesättigten Verbindungen der allgemeinen Formel (IV) im großen Überschuß als Verdünnungsmittel eingesetzt werden.

Die Reaktionstemperaturen liegen vorzugsweise bei 0 bis 50°C, insbesondere zwischen 10 und 40°C.

Bei der Durchführung des Verfahrens B) setzt man in der Regel 1 Mol der Verbindung (II) mit 2 bis 15 Mol, vorzugsweise mit 6 bis 10 Mol der Verbindung (IV) in Gegenwart von 0,05 bis 0,2 Mol des Katalysators um. Die Reaktionsdauer ist von der Temperatur abhängig und liegt z.B. bei 5 bis 20 Stunden.

Falls erforderlich, kann eine Reinigung durch Destillation,

Umkristallisation oder durch Chromatographie erfolgen. Für den Fall, daß Verbindungen mit der allgemeinen Formel (V) erhalten werden, werden diese anschließend in bekannter Weise (beispielsweise sauer) verseift. Zur sauren Verseifung eignen sich starke anorganische Säuren wie Salzsäure oder Schwefelsäure. Bei der Verwendung von Schwefelsäure ist es vorteilhaft, zunächst mit konz. Schwefelsäure bei etwa Raumtemperatur bis zur Stufe des Amids zu verseifen und anschließend in einer zweiten Stufe mit verdünnter Schwefelsäure bei erhöhter Temperatur die Verseifung zur freien Säure durchzuführen. Dabei ist es nicht erforderlich, die Stufe des Amids zu isolieren.

Als für die erfindungsgemäßen Arzneimittel geeignete Wirkstoffe seien im einzelnen genannt:
4-(Thiazol-2-yl)thio-butansäure,
4-(4-Methyl-thiazol-2-yl)thio-butansäure,
4-(4-Methyl-5-acetyl-thiazol-2-yl)thio-butansäure,
4-(4,5-Dimethyl-thiazol-2-yl)thio-butansäure,
3-(4,5-Dichlor-thiazol-2-yl)thio-propansäure,
4-(4,5-Dichlor-thiazol-2-yl)thio-butansäure,
4-(4,5-Dichlor-thiazol-2-yl)thio-butansäuremethylester,
5-(4,5-Dichlor-thiazol-2-yl)thio-pentansäure,
4-(4,5-Dicyano-thiazol-2-yl)-thio-butansäure,
4-(4-Chlor-5-cyano-thiazol-2-yl)-thio-butansäure
4-(5-Amino-thiazol-2-yl)thio-butansäure,
4-(5-Trifluormethyl-thiazol-2-yl)thio-butansäure,
4-(4,5-Diphenyl-thiazol-2-yl)thio-butansäure,
4-[4,5-Bis(4-methoxyphenyl)-thiazol-2-yl]thio-butansäure,
4-[4,5-Bis(3,4-methylendioxyphenyl)-thiazol-2-yl]thio-butansäure,
4-(Benzthiazol-2-yl)thio-butansäure,

4-(Benzthiazol-2-yl)thio-butansäuremethylester,
5-(Benzthiazol-2-yl)thio-pentansäure,
3-(Benzthiazol-2-yl)thio-propansäure,
4-(5-Chlor-benzthiazol-2-yl)thio-butansäure,
4-(6-Fluor-benzthiazol-2-yl)thio-butansäure,
4-(4-Fluor-benzthiazol-2-yl)-thio-butansäure,
4-(5-Fluor-benzthiazol-2-yl)thio-butansäure,
4-(7-Fluor-benzothiazol-2-yl)thio-butansäure,
4-(5,6-Difluor-benzthiazol-2-yl)-thio-butansäure,
4-(4-Ethoxy-6-chlor-benzthiazol-2-yl)thio-butansäure,
4-(5-Ethoxy-benzthiazol-2-yl)thio-butansäure,
4-(6-Dimethylamino-benzthiazol-2-yl)thio-butansäure,
4-(6-Amino-benzthiazol-2-yl)thio-butansäure,
4-(6-Nitro-benzthiazol-2-yl)thio-butansäure,
4-(5-Carboxy-benzthiazol-2-yl)thio-butansäure,
4-(6-Formamido-benzthiazol-2-yl)thio-butansäure,
4-(4,5,6,7-Tetrahydro-benzthiazol-2-yl)thio-butansäure,
4-(5,6-Dihydro-4H-cyclopentathiazol-2-yl)thio-butansäure.

Als Zubereitungsformen kommen die üblichen galenischen Applikationsformen in Frage, beispielsweise Cremes, Tabletten, Pillen, Kapseln, Suppositorien, Emulsionen, Infusions- und Injektionslösungen. Diese Zubereitungsformen werden nach an sich bekannten Methoden hergestellt unter Verwendung üblicher Hilfs- und Trägerstoffe.

Der Einsatz der so hergestellten Arzneimittel erfolgt je nach Bedarf z.B. durch lokale, parenterale oder orale Verabreichung.

Besonders geeignet sind Formulierungen, die die erfindungsgemäßen Verbindungen in Konzentrationen von etwa

o,1 bis 10 Gew.-% enthalten. Besonders bevorzugt sind wäßrige Lösungen, die gegebenenfalls auf einen pH-Wert von 6 bis 8 gepuffert sind.

Die Dosierung der Thiazolderivate in den erfindungsgemäßen Arzneimitteln liegt vorzugsweise in einem Bereich von 0,05 bis 100 mg/kg, insbesondere von 0,1 bis 20 mg/kg Körpergewicht.

Die in den erfindungsgemäßen Arzneimitteln enthaltenen 2-Mercaptothiazol-Derivate sind also Thromboxan-Antagonisten und Plättchenaggregationshemmer zur Verhütung und Behandlung von Ischämien, insbesondere von Herzinfarkten nach Myokardischämien, Angina Pectoris, thromboembolischen Erkrankungen im venösen und arteriellen Bereich, peripheren Durchblutungsstörungen, allergischen und asthmatischen Erkrankungen, Lungenembolien und Oedemen, insbesondere Lungenoedemen, von pulmonaler Hypertension und Migräne geeignet.

Die in den nachstehenden Ausführungsbeispielen beschriebenen pharmakologischen Wirkungen der erfindungsgemäßen Arzneimittel wurden nach folgenden Methoden untersucht:

<u>Gefäßkonstriktorische Wirkung</u>

Die gefäßkonstriktorische Wirkung von Thromboxan wird mit Hilfe der isometrischen Kontraktion von Kaninchenaortenstreifen gemessen (Furchtgott, R. F; Bhadrakom, S. J., Pharmacol. Exp. Ther. <u>108</u>, 129 - 143, 1953). Männliche

Kaninchen (Russen, Gewicht 1,8 - 2 kg) werden mit Pentobarbital getötet. Die Brustaorta wird entnommen und spiralförmig aufgeschnitten. Die Streifen (Breite 2 - 3 mm, Länge 3 cm) werden unter einer Spannung von 2 g in 10 ml - Organbädern, die Carbogen durchperlten Krebs-Henseleit-Puffer von 37°C enthalten, suspendiert. Zur Erhöhung der Spezifität der Methode enthält der Puffer folgende Inhibitoren (jeweils 100 µg/l): Propanololhydrochlorid, Phentolamin, Cyproheptadin, Atropin, Mebhydrolin und Indometacin. Die Kontraktionen werden nach Erreichen eines konstanten Basaltonus durch $TXA_2$, generiert aus gewaschenen Human-Thrombozyten, ausgelöst.

Zur Präparation der Thrombozytensuspension wird Humanblut in 1 %iger EDTA-Lösung aufgenommen und bei 150 G 20 Minuten lang zentrifugiert. Das plättchenreiche Plasma wird abgehebert und bei 750 G für 20 Minuten zentrifugiert. Das plättchenarme Plasma wird abdekantiert und die verbleibenden Plättchen in Puffer (0,15 M NaCl, 0,15 M Tris/HCl pH 7.4, 77 mM EDTA; 90 : 8 : 2) aufgenommen.

Die Thromboxan-Generation erfolgt durch Stimulation der Plättchen mit Thrombin bei 37°C unter Rühren. Nach 30 sec wird die Plättchensuspension zu dem mit Prüfsubstanz bzw. Lösungsmittel vorbehandelten Aortenstreifen gegeben und damit die Kontraktionen ausgelöst. Die prozentuale Hemmung errechnet sich aus den entsprechenden Ausschlägen.

Thrombocytenaggregationshemmung

Für die Bestimmung der thrombocytenaggregationshemmenden Wirkung wird Blut von gesunden Spendern, die mindestens 14 Tage lang kein Medikament eingenommen hatten, verwendet. Das Blut wird in 3.8 %iger Natriumcitratlösung aufgenommen. Plättchenreiches Plasma (PRP) wird durch 20 min. lange Zentrifugation bei 150 G bei Raumtemperatur gewonnen (Jürgens/Beller: Klinische Methoden der Blutgerinnungsanalyse; Thieme-Verlag, Stuttgart 1959). Die Plättchenaggregation wird nach der turbidometrischen Methode (Born, G. V. R.: J. Physiol. 162 67, 1962) im Aggregometer bei 37°C bestimmt. Hierzu wird PRP mit Prüfsubstanz bei 37°C inkubiert und anschließend die Aggregation durch Zugabe einer Kollagensuspension ausgelöst. Die prozentuale Hemmung errechnet sich aus den entsprechenden Ausschlägen.

Ergebnisse

An den in der folgenden Tabelle 1 genannten Verbindungen wurde die spezifische Hemmung der durch Thromboxan induzierten Gefäßkontraktionen bestimmt.

Tabelle 1

| Verbindung gemäß Beispiel Nr. | Konzentration (g/ml) | Hemmung (%) ($\bar{x}$ ± SEM) |
|---|---|---|
| 21 | $3 \times 10^{-5}$ | 59 ± 6 |
| 9 | $1 \times 10^{-4}$ | 100 ± 0 |
| | $3 \times 10^{-5}$ | 84 ± 3 |
| | $1 \times 10^{-5}$ | 58 ± 7 |
| | $3 \times 10^{-6}$ | 34 ± 6 |
| | $1 \times 10^{-6}$ | 16 ± 7 |
| 25 | $3 \times 10^{-5}$ | 31 ± 18 |
| 23 | $3 \times 10^{-5}$ | 47 ± 10 |
| 20 | $3 \times 10^{-5}$ | 62 ± 7 |
| 17 | $3 \times 10^{-5}$ | 32 ± 8 |
| 11 | $3 \times 10^{-5}$ | 71 ± 4 |
| 11a | $3 \times 10^{-5}$ | 82 ± 1 |
| 12 | $3 \times 10^{-5}$ | 85 ± 5 |
| 12a | $3 \times 10^{-5}$ | 78 ± 1 |

An den in der folgenden Tabelle 2 genannten Verbindungen wurde die Hemmung der Kollagen-induzierten Thrombozytenaggregation untersucht.

Tabelle 2

| Verbindung gemäß Beispiel Nr. | Konzentration (g/ml) | Hemmung (%) ($\bar{x} \pm$ SEM) |
|---|---|---|
| 21 | $3 \times 10^{-5}$ | 69 |
| 9 | $3 \times 10^{-5}$ | 52 |

Die erfindungsgemäß zu verwendenden 2-Mercaptothiazolderivate weisen folgende Vorteile gegenüber bekannten $TXA_2$-Antagonisten von nicht prostanoider Struktur auf: Die Verbindungen hemmen spezifisch durch Thromboxan bzw. durch Thromboxan- und Endoperoxid-Analoga induzierte Spasmen und sind unwirksam gegenüber anderen Antagonisten ($K^+$, Serotonin, Noradrenalin). Sie sind dabei stärker wirksam als die bekannten $TXA_2$-Hemmer Phthalazinol und Pyridinolcarbamat (Shimamoto, T., Y. Takashima, M. Kobayashi, K. Moriya und T. Takahashi. Proc. Jap. Academy 52, 591, 1976). Phthalazinol wirkt zudem nicht als spezifischer $TXA_2$-Antagonist (Tanaka, K., Harada, Y., Katori, M. Prostaglandins 17, 235, 1979).

In den nachstehenden Herstellungsbeispielen wird die Synthese einiger erfindungsgemäßer bzw. in den erfindungsgemäßen Arzneimitteln zu verwendender 2-Mercaptothiazolderivate beschrieben.

Beispiel 1

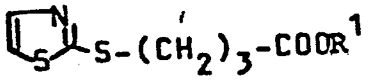

($R^1$ = H; $CH_3$)

4-(Thiazol-2-yl)thio-butansäure(methylester)

23,4 g (0,2 Mol) 2-Mercaptothiazol werden in 100 ml Acetonitril gelöst und bei 15 bis 20°C mit einer Lösung von 33,4 g (0,22 Mol) 1,8-Diazabicyclo[5.4.0]undec-7-en (DBU) in 30 ml Acetonitril versetzt. Man rührt das Gemisch 10 Minuten bei 25°C, versetzt es anschließend bei 25 bis 30°C mit einer Lösung von 39,8 g (0,22 Mol) 4-Brombutansäuremethylester in 20 ml Acetonitril und erwärmt 1 Stunde auf 75 bis 80°C. Dann wird das Lösungsmittel im Vakuum abdestilliert und der Destillationsrückstand zwischen Wasser und Ethylacetat verteilt. Die organische Phase wird mit Wasser gewaschen und über Natriumsulfat getrocknet. Man entfernt das Lösungsmittel im Vakuum und destilliert den Rückstand im Hochvakuum. Man erhält auf diese Weise 41,8 g (96 % Ausbeute) 4-(Thiazol-2-yl)thiobutansäuremethylester. $Kp_{0,008}$ 130 - 140°C (Lufttemperatur).

10,85 g (0,05 Mol) 4-(Thiazol-2-yl)thio-butansäure-methylester werden unter Stickstoff in 55 ml 1 n ethanolisch-wäßriger (94 % Ethanol, 6 % Wasser) Kalilauge gelöst und bei 25°C 12 Stunden stehen gelassen. Man entfernt das

Lösungsmittel im Vakuum, nimmt den Rückstand in Wasser auf, säuert bei 10 bis 20°C die wäßrige Lösung bis zu einem pH von 4 an, saugt den Niederschlag ab und trocknet ihn nach einmaligem Waschen mit Wasser über Kaliumhydroxid. Man erhält auf diese Weise 9,2 g (90 % Ausbeute) 4-(Thiazol-2-yl)thio-butansäure. Fp 57°C.

Beispiele 2 - 21

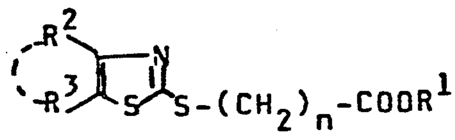

Analog Beispiel 1 erhält man die in der Tabelle 3 aufgeführten Verbindungen.

Tabelle 3

| Beisp. Nr. | $R^1$ | $R^2$ | $R^3$ | n | Ausbeute (%) | Fp °C (Kp) | Verwendet. Lösungsmittel in Stufe 1 | $R_F$ (Kieselgel) |
|---|---|---|---|---|---|---|---|---|
| 2 | H | $CH_3$ | H | 3 | 81 | - | Acetonitril | 0,21 C:E:S = 60:40:1 1) |
| 3 | H | $CH_3$ | $COCH_3$ | 3 | 61 | 93 | DMF 2) | 0,14 C:E:S = 60:40:1 |
| 4 | H | $CH_3$ | $CH_3$ | 3 | 80 | 59 | DMF | 0,14 C:E:S = 60:40:1 |
| 5 | H | Phenyl | H | 3 | 61 | 87 | DMF | 0,15 C:E:S = 75:25:1 |
| 6 | H | Phenyl | Phenyl | 3 | 70 | 120 | DMF | 0,14 C:E:S = 75:25:1 |
| 7 | H | $CH_3O$- 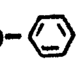 - | $CH_3O$- 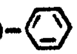 - | 3 | 91 | 175 | DMF | 0,13 C:E:S = 75:25:1 |
| 21 | H | Cl | Cl | 3 | 60 | - | DMF | 0,21 C:E:S = 60:40:1 |
| | | $R^2 + R^3$ | | | | | | |
| 8 | $CH_3$ | 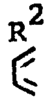 | | 3 | 89 | (190/0,01 mb) | DMF | 0,115 $CH_2Cl_2$:C = 90:10 |
| 9 | H | 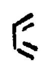 | | 3 | 90 | 104 | - | 0,12 C:E:S = 75:25:1 |
| 10 | H | 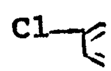 | | 3 | 86 | 122 | DMF | 0,25 C:E:S = 60:40:1 |
| 11 | H | 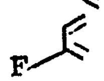 | | 3 | 85 | 103 | DMF | 0,11 C:E:S = 75:25:1 |
| 11a | H | 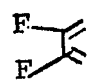 | | 3 | 82 | 127 | DMF | 0,11 C:E:S = 75:25:1 |

**Tabelle 3** (Fortsetzung)

| Beisp. Nr. | $R^1$ | $R^2 + R^3$ | n | Ausbeute (%) | Fp °C | verwendetes Lösungsmittel in Stufe 1 | $R_F$ (Kieselgel) |
|---|---|---|---|---|---|---|---|
| 12 | H | 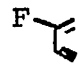 | 3 | 94 | 98 | DMF | 0,11 C:E:S = 75:25:1 |
| 12a | H | 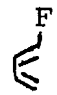 | 3 | 88 | 116 | DMF | 0,12 C:E:S = 75:25:1 |
| 13 | H | 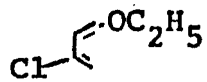 | 3 | 80 | 145 | DMF | 0,19 C:E:S = 60:40:1 |
| 14 | H | 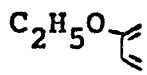 | 3 | 83 | 83 | DMF | 0,19 C:E:S = 60:40:1 |
| 15 | H | 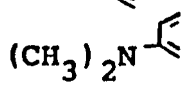 | 3 | 75 | 84 | DMF | 0,21 C:E:S = 60:40:1 |
| 16 | H | 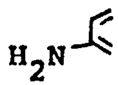 | 3 | 34 | 149 | DMF | 0,07 C:E:S = 60:40:1 |
| 17 | H | 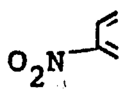 | 3 | 41 | 131 | DMF | 0,10 C:E:S = 75:25:1 |
| 18 | H | 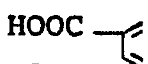 | 3 | 40 | 228 | DMF | 0,14 C:E:S = 40:60:1 |
| 19 | H | 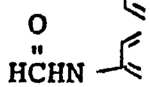 | 3 | 57 | 172 | DMF | 0,13 C:E:S = 40:60:1 |
| 20 | H | 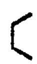 | 3 | 82 | - | DMF | 0,24 C:E:S = 60:40:1 |

1) C = Cyclohexan; E = Ethylacetat; S = Essigsäure; 2) DMF = Dimethylformamid

Beispiel 22

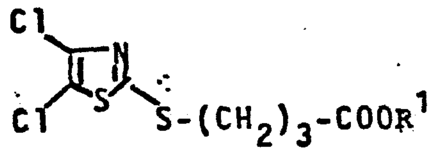

($R^1$ = H; $CH_3$)

4-(4,5-Dichlor-thiazol-2-yl)thio-butansäure(methylester)

18,6 g (0,1 Mol) 4,5-Dichlor-2-mercaptothiazol werden in 100 ml Dimethylformamid gelöst und bei 5 bis 10°C innerhalb von 30 Minuten mit 3,0 g 80 %igem Natriumhydrid versetzt. Man rührt noch eine Stunde bei 25°C, versetzt bei 20 bis 25°C mit einer Lösung von 18,1 g (0,1 Mol) Brombutansäuremethylester in 10 ml Dimethylformamid, erwärmt 6 Stunden bei 80 bis 85°C, entfernt anschließend das Lösungsmittel im Vakuum und verteilt den Rückstand zwischen Ethylacetat und Wasser. Die organische Phase wird mit Wasser gewaschen und über Natriumsulfat getrocknet. Man entfernt das Lösungsmittel im Vakuum, reinigt den Rückstand durch Chromatographie an Kieselgel mit einem Methylenchlorid-Cyclohexan-Gemisch (80 Volumenteile + 20 Volumenteile) und erhält auf diese Weise 12,0 g (42 %Ausbeute) 4-(4,5-Dichlor-thiazol-2-yl)-thio-butansäuremethylester. $R_F$ 0.30 (gleiches Laufmittelgemisch).

9,8 g (0,34 Mol) 4-(4,5-Dichlor-thiazol-2-yl)thio-butansäuremethylester werden unter Stickstoff in 34,2 ml 2 n ethanolisch-wäßriger (90 % Ethanol, 10 % Wasser) Kalilauge gelöst und bei 25°C 12 Stunden stehen gelassen. Man entfernt das Lösungsmittel im Vakuum, nimmt den Rückstand in Wasser auf, säuert bei 10 bis 20°C die wäßrige Lösung bis zu einem pH von 4 an, extrahiert die ölig anfallende Säure mit Ethylacetat, gewinnt sie aus dieser Lösung durch Abdampfen des Ethylacetats im Vakuum und reinigt sie durch Chromatographie an Kieselgel mit einem Gemisch von 60 Volumenteilen Cyclohexan, 40 Volumenteilen Ethylacetat und 1 Volumenteil Essigsäure. Man erhält auf diese Weise 7,7 g (83 % Ausbeute) 4-(4,5-Dichlor-thiazol-2-yl)thio-butansäure. $R_F$ 0,29 (gleiches Laufmittel).

Beispiele 23 - 25

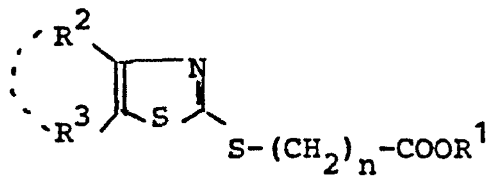

Analog Beispiel 22 erhält man die in Tabelle 4 aufgeführten Verbindungen.

Tabelle 4

| Beisp. Nr. | $R^1$ | $R^2$ | $R^3$ | n | Ausbeute (%) | Fp °C | verwendetes Lösungsmittel in Stufe 1 | $R_F$ (Kieselgel) |
|---|---|---|---|---|---|---|---|---|
| 23 | H | Cl | Cl | 4 | 20 | - | DMF[2)] | 0,15 C:E:S = 80:20:1[1)] |
| 24 | $CH_3$ | $R^2$ + $R^3$ 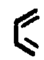 | | 3 | 64 | - | DMF | 0,15 $CH_2Cl_2$:C = 90 :10 |
| 25 | H | 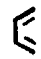 | | 4 | 35 | 82 | DMF | 0,20 C:E:S = 75:25:1 |

1) C = Cyclohexan; E = Ethylacetat; S = Essigsäure; 2) DMF = Dimethylformamid;

Beispiel 26

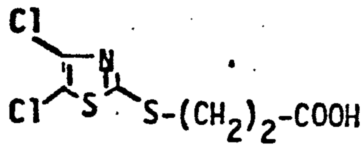

3-(4,5-Dichlor-thiazol-2-yl)thio-propansäure

9,3 g (0,05 Mol) 4,5-Dichlor-2-mercaptothiazol werden in 25 ml Acrylnitril suspendiert und bei 20 bis 25°C mit 0,5 g N-Benzyl-N,N-dimethyl-aniliniumhydroxid versetzt. Man rührt 24 Stunden bei 25°C, entfernt anschließend das überschüssige Acrylnitril im Vakuum, nimmt den Rückstand in Methylenchlorid auf, trennt unlösliche Anteile durch Filtration ab, entfernt das Lösungsmittel durch Destillation, und reinigt den Rückstand durch Chromatographie an Kieselgel mit einem Gemisch von 60 Volumenteilen Methylenchlorid und 40 Volumenteilen Cyclohexan. Man erhält auf diese Weise 6,3 g (53 %) 3-(4,5-Dichlor-thiazol-2-yl)thiopropansäurenitril. $R_f$ 0,22 (gleiches Laufmittel).

5,4 g (0,023 Mol) 3-(4,5-Dichlor-thiazol-2-yl)thiopropansäurenitril werden in 16,5 ml konz. Schwefelsäure bei 0 bis 10°C gelöst. Man läßt das Gemisch 24 Stunden bei 25°C stehen, verdünnt anschließend unter Eiskühlung mit 13 ml Wasser, rührt das Reaktionsgemisch 30 Minuten bei 95 bis 100°C und kühlt dann auf 25°C ab. Es wird mit Wasser verdünnt, zweimal mit Ethylacetat extrahiert, die Ethylacetatlösung mit Wasser gewaschen, über Natriumsulfat getrocknet und im Vakuum eingedampft. Der Rückstand wird an Kieselgel mit einem Gemisch von 75 Volumenteilen Cyclohexan, 25 Volumenteilen Ethylacetat und 1 Volumenteil Essigsäure gereinigt. Man erhält auf diese Weise 4,3 g (74 % Ausbeute) 3-(4,5-Dichlor-thiazol-2-yl)thio-propansäure.
Fp 104°C

| | | %C | %H | %N | %Cl |
|---|---|---|---|---|---|
| Analyse: $C_6H_5Cl_2NO_2S_2$ | | | | | |
| (Mol 258,2) | ber. | 27,91 | 1,95 | 5,43 | 27,47 |
| | gef. | 27,85 | 2,10 | 5,45 | 27,15 |

Patentansprüche

1. Thromboxan-antagonistische und plättchenaggregationshemmende Arzneimittel, dadurch gekennzeichnet, daß sie 2-Mercaptothiazolderivate der allgemeinen Formel

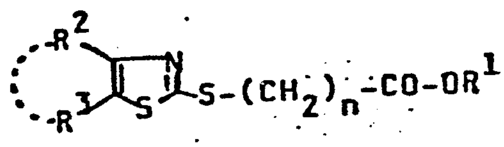

(I)

oder deren pharmakologisch unbedenkliche Salze enthalten, in welcher

$R^1$ für Wasserstoff oder einen gegebenenfalls verzweigten Alkylrest mit 1 bis 6 C-Atomen steht,

n eine ganze Zahl von 2 bis 10 bedeutet, und

$R^2$ und $R^3$ gleich oder verschieden sind und für Wasserstoff, Halogen, Cyano, Amino, einen gegebenenfalls verzweigten und gegebenenfalls durch Halogen, Hydroxy, Methoxy oder Carboxy substituierten Alkyl- oder Acylrest mit 1 bis 6 C-Atomen oder einen Arylrest mit 6 bis 10 C-Atomen, welcher gegebenenfalls durch Halogen, $CF_3$, Nitro, $C_1$-$C_6$-Alkyl, $C_1$-$C_6$-Alkoxy, Carboxy, Amino, Alkyl-

amino oder Dialkylamino mit 1 bis 4 C-Atomen in den Alkylgruppen, Acyl mit 1 bis 6 C-Atomen oder Acylamino mit 1 bis 6 C-Atomen oder einen Methylendioxyrest substituiert ist, stehen oder

$R^2$ und $R^3$ zusammen mit dem Thiazolring einen gegebenenfalls teilweise hydrierten aromatischen Ring mit 5 bis 7 C-Atomen bilden, welcher gegebenenfalls durch 1 oder 2 Substituenten aus der Gruppe Halogen, Hydroxy, Alkoxy mit 1 bis 6 C-Atomen, Amino, Alkylamino oder Dialkylamino mit 1 bis 4 C-Atomen in den Alkylgruppen, Carboxy, Nitro, Alkyl mit 1 bis 6 C-Atomen, Trifluormethyl, Acyl mit 1 bis 6 C-Atomen oder Acylamino mit 1 bis 6 C-Atomen, oder durch einen Methylendioxyrest substituiert ist.

2. Arzneimittel nach Anspruch 1, dadurch gekennzeichnet, daß $R^1$ für Wasserstoff oder Methyl steht.

3) Arzneimittel nach Anspruch 1 oder 2, dadurch gekennzeichnet, daß n eine ganze Zahl von 2 bis 4 bedeutet.

4) Arzneimittel nach Anspruch 1 bis 3, dadurch gekennzeichnet, daß

$R^2$ und $R^3$ gleich oder verschieden sind und für Wasserstoff, Chlor, Brom, Cyano, Acetyl, einen gegebenenfalls verzweigten Alkylrest mit 1 bis 6 C-Atomen, welcher gegebenenfalls durch Halogen substituiert ist, oder einen Phenylrest stehen, welcher gegebenenfalls durch Halogen, Trifluormethyl, Hydroxy, $C_1$-$C_4$-Alkoxy, Nitro oder einen Methylendioxyrest substituiert ist, oder

$R^2$ und $R^3$ zusammen für einen Propylen-, Butylen- oder Pentylenrest stehen oder zusammen mit dem Thiazolring einen aromatischen 6-gliedrigen Ring bilden, welcher gegebenenfalls durch Halogen, Hydroxy, $C_1$-$C_4$-Alkoxy, Amino, Dialkylamino, Nitro oder Carboxy substituiert ist.

5) Verwendung von Verbindungen gemäß Formel (I) von Anspruch 1 bei der Bekämpfung von thromboembolischen, aterosklerotischen, ischämischen, asthmatischen und anderen unter Thromboxanbeteiligung ablaufenden Erkrankungen.

6) Verbindungen der allgemeinen Formel

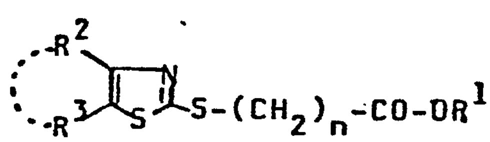

oder deren pharmakologisch unbedenkliche Salze, in welcher

$R^1$ für Wasserstoff oder einen gegebenenfalls verzweigten Alkylrest mit 1 bis 6 C-Atomen steht,

n eine ganze Zahl von 2 bis 10 bedeutet und

$R^2$ und $R^3$ gleich oder verschieden sind und für Wasserstoff, Halogen, Cyano,Amino, einen gegebenenfalls verzweigten und gegebenenfalls durch Halogen, Hydroxy, Methoxy oder Carboxy substituierten Alkyl- oder Acylrest mit 1 bis 6 C-Atomen oder einen Arylrest mit 6 bis 10 C-Atomen, welcher gegebenenfalls durch Halogen, $CF_3$, Nitro, $C_1$-$C_6$-Alkyl, $C_1$-$C_6$-Alkoxy, Carboxy, Amino, Alkylamino oder Dialkylamino mit 1 bis 4 C-Atomen in den Alkylgruppen, Acyl mit 1 bis 6 C-Atomen oder Acylamino mit 1 bis 6 C-Atomen oder einen Methylendioxyrest substituiert ist, stehen oder

$R^2$ und $R^3$ zusammen mit dem Thiazolring einen gegebenenfalls teilweise hydrierten aromatischen Ring mit 5 bis 7 C-Atomen bilden, welcher gegebenenfalls durch 1 oder 2 Substituenten aus der Gruppe Halogen, Hydroxy, Alkoxy mit 1 bis 6 C-Atomen,

Amino, Alkylamino oder Dialkylamino mit 1 bis 4 C-Atomen in den Alkylgruppen, Carboxy, Nitro, Alkyl mit 1 bis 6 C-Atomen, Trifluormethyl, Acyl mit 1 bis 6 C-Atomen oder Acylamino mit 1 bis 6 C-Atomen, oder durch einen Methylendioxyrest substituiert ist,

mit der Maßgabe, daß $R^2$ und $R^3$ zusammen mit dem Thiazolring keinen unsubstituierten 6-gliedrigen aromatischen Ring bilden, wenn $R^1$ für Wasserstoff steht.

7) Verbindungen der Formel

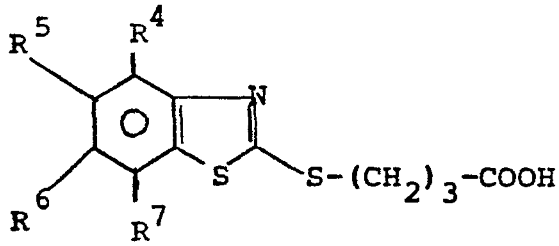

wobei
einer oder zwei der Reste $R^4$, $R^5$, $R^6$ und $R^7$ für Fluor und die anderen für Wasserstoff stehen.

8) Verbindungen der Formel

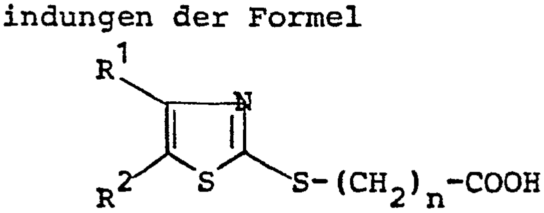

wobei

0137426

n 3 oder 4 bedeutet und
$R^1$ und $R^2$ beide für Chlor oder zusammen für eine Tetramethylengruppe stehen.

9) Arzneimittel, gekennzeichnet durch den Gehalt an einer Verbindung gemäß Anspruch 7 oder 8.

10) Verfahren zur Herstellung von Verbindungen gemäß Anspruch 6, dadurch gekennzeichnet, daß man

A) 2-Mercaptothiazole der allgemeinen Formel (II)

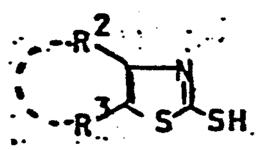

(II)

in welcher
$R^2$ und $R^3$ gleich oder verschieden sind und für Wasserstoff, Halogen, Cyano, Amino, einen gegbenenfalls verzweigten und gegebenenfalls durch Halogen, Hydroxy, Methoxy oder Carboxy substituierten Alkyl- oder Acylrest mit 1 bis 6 C-Atomen oder einen Arylrest mit 6 bis 10 C-Atomen, welcher gegebenenfalls durch Halogen, $CF_3$, Nitro, $C_1$-$C_6$-Alkyl, $C_1$-$C_6$-Alkoxy, Carboxy, Amino, Alkylamino oder Dialkylamino mit 1 bis 4 C-Atomen in den Alkylgruppen, Acyl mit 1 bis

6 C-Atomen oder Acylamino mit 1 bis 6 C-Atomen oder einen Methylendioxyrest substituiert ist, stehen oder

$R^2$ und $R^3$ zusammen mit dem Thiazolring einen gegebenenfalls teilweise hydrierten aromatischen Ring mit 5 bis 7 C-Atomen bilden, welcher gegebenenfalls durch 1 oder 2 Substituenten aus der Gruppe Halogen, Hydroxy, Alkoxy mit 1 bis 6 C-Atomen, Amino, Alkylamino oder Dialkylamino mit 1 bis 4 C-Atomen in den Alkylgruppen, Carboxy, Nitro, Alkyl mit 1 bis 6 C-Atomen, Trifluormethyl, Acyl mit 1 bis 6 C-Atomen oder Acylamino mit 1 bis 6 C-Atomen, oder durch einen Methylendioxyrest substituiert ist,

mit $\omega$ -Halogencarbonsäureestern der allgemeinen Formel (III)

$$Hal-(CH_2)_n-CO-OR^1 \qquad (III)$$

in welcher

$R^1$ für einen gegebenenfalls verzweigten Alkylrest mit 1 bis 6 C-Atomen steht,

Hal ein Halogenatom bedeutet,

n eine ganze Zahl von 2 bis 10 bedeutet,

in Gegenwart einer Base und gegebenenfalls inerten organischen Lösungsmitteln bei Temperaturen zwischen

0137426

0 und 150°C umsetzt und gegebenenfalls die erhaltenen Ester zur freien Säure verseift

oder

B) für den Fall, daß n für die Zahl 2 steht, 2-Mercaptothiazole der allgemeinen Formel (II) mit Vinylverbindungen der allgemeinen Formel (IV)

$$CH_2=CH-R^4 \qquad (IV)$$

in welcher
$R^4$ für CN oder $CO-OR^1$ steht,

in Gegenwart eines basischen Katalysators und gegebenenfalls in einem inerten organischen Lösungsmittel bei Temperaturen zwischen 0 und 80°C umsetzt und für den Fall, daß $R^4$ für $CO-OR^1$ steht, die so erhaltenen Ester gegebenenfalls verseift und für den Fall, daß $R^4$ für CN steht, die erhaltene Verbindung mit der allgemeinen Formel (V)

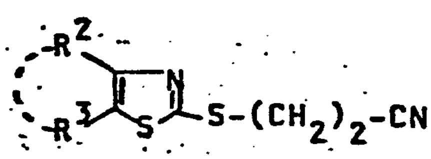

(V)

verseift.